# EUROPEAN PATENT SPECIFICATION

(11) **EP 1 880 988 B1**
(45) Date of publication and mention of the grant of the patent: **21.12.2011**
(21) Application number: 06730826.2
(22) Date of filing: 31.03.2006
(51) Int. Cl.: C07C 41/26, C07C 43/13

(54) **PROCESS FOR PRODUCING GLYCERYL ETHER**
VERFAHREN ZUR HERSTELLUNG VON GLYCERYLETHER
PROCEDE DE FABRICATION DE GLYCERYL ETHER

(30) Priority: 01.04.2005 JP 2005106587; 28.12.2005 JP 2005379439
(43) Date of publication of application: 23.01.2008
(73) Proprietor: Kao Corporation, Chuo-ku Tokyo 103-8210 (JP)
(72) Inventor: SAITO, Akira, Wakayama 640-8580 (JP); NAKA, Kenichi, Wakayama 640-8580 (JP); SHIRASAWA, Takeshi, Wakayama 640-8580 (JP)
(74) Representative: HOFFMANN EITLE
(86) International application number: PCT/JP2006/306878
(87) International publication number: WO 2006/106940

(56) References cited:
- JP-A- 2002 088 000
- JP-A- 2003 267 901

## Description

### TECHNICAL FIELD

The present invention relates to a method for producing a glyceryl ether.

### BACKGROUND ART

A glyceryl ether obtainable by hydrolyzing a glycidyl ether is a compound useful as, for example, a solvent, an emulsifying agent, a dispersant, a detergent, a foaming agent, or the like.

In general, a glyceryl ether is produced in the presence of a catalyst; however, as a method capable of producing a glyceryl ether in the absence of a catalyst, for example, a method including the step of hydrolyzing a glycidyl ether with a water in a subcritical state has been known (Patent Publication 1).
Patent Publication 1: JP 2002-88000 A
JP 2003 267901 A discloses a continuous hydrolysis method, wherein reaction water is recycled.

### SUMMARY OF THE INVENTION

Specifically, the present invention relates to:
a method for producing a glyceryl ether, including the steps of feeding a compound represented by the general formula (I): 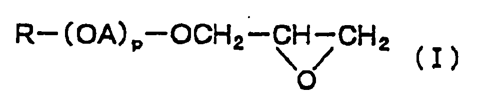 wherein R is a hydrocarbon group having 1 to 20 carbon atoms, a part or all of the hydrogen atoms of which may be substituted with a fluorine atom; OA, which may be identical or different, is an oxyalkylene group having 2 to 4 carbon atoms; and p is the number of from 0 to 20,
   and water to a reactor, and subjecting the compound to a hydrolytic reaction under conditions that water is in a subcritical state or a supercritical state,
   wherein the method includes the step of:
   (1) collecting water from a reaction mixture after the hydrolytic reaction, adjusting a pH of the water to to a pH ranging from 3.5 to 12 , and feeding the collected water to the reactor; or
   (2) subjecting water collected from a reaction mixture after the hydrolytic reaction to a filtration treatment with a hydrophilically treated reverse osmosis membrane, and feeding the resulting treated water as a recycled water to the reactor; and
a method for preventing coloration of a glyceryl ether, produced by the steps of:
   (A) feeding a compound represented by the general formula (I): 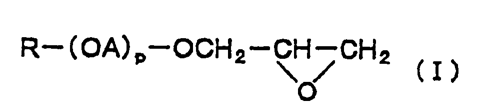 wherein R is a hydrocarbon group having 1 to 20 carbon atoms, a part or all of the hydrogen atoms of which may be substituted with a fluorine atom; OA, which may be identical or different, is an oxyalkylene group having 2 to 4 carbon atoms; and p is the number of from 0 to 20, and water to a reactor, and subjecting the compound to a hydrolytic reaction under conditions that water is in a subcritical state or a supercritical state, and
   (B) collecting water from a reaction mixture after the hydrolytic reaction, and feeding the collected water to the reactor,
      wherein the method includes the step, in the step (B), of:
      (B-1) adjusting a pH of the water to a pH ranging from 3.5 to 12 , and feeding the collected water to the reactor, or
      (B-2) subjecting water collected from a reaction mixture after the hydrolytic reaction to a filtration treatment with a hydrophilically treated reverse osmosis membrane, and feeding the resulting treated water as a recycled water to the reactor.

### DETAILED DESCRIPTION OF THE INVENTION

However, in the method mentioned above, in a case of collecting water from a reaction mixture after a hydrolytic reaction, feeding the water to a reactor in which the hydrolytic reaction is carried out, and further using the water for a hydrolytic reaction, for the purpose of using a large amount of the water in the hydrolytic reaction while reducing loss of water and maintaining a reaction selectivity at a high level, some disadvantages in which the quality of the resulting glyceryl ether is lowered, such as the formation of by-products other than the glyceryl ethers is increased with the passage of time, and a hue of the glyceryl ether is worsened have been found.

Specifically, the present invention relates to provide a method for efficiently producing a high-quality glyceryl ether, which is capable of reducing loss of water to be used, and controlling the formation of by-products, thereby being capable of preventing the worsening of a hue of the glyceryl ether.

The present inventors have found that there is a correlation between an increase in the by-products as mentioned above and lowering of a pH of water usable in a hydrolytic reaction, so that the formation of by-products during the reaction can be controlled by adjusting a pH of water; moreover, the present inventors have found that, with unknown reasons, even when the hydrolytic reaction of a glycidyl ether is repeated by using a water obtained by subjecting a water collected from a reaction mixture to a filtration treatment with a reverse osmosis membrane, the color of the glyceryl ether is lowered, so that a high-quality glyceryl ether is obtained. The present invention has been perfected thereby.

According to the present invention, loss of water to be used can be reduced, and the formation of by-products can be controlled, thereby preventing a hue of the glyceryl ether from being worsened, whereby a high-quality glyceryl ether can be efficiently produced.

The present invention is a method for producing a glyceryl ether in which a given glycidyl ether is used as a raw material, including the steps of feeding the raw material and water to a reactor, and subjecting the raw material to a hydrolytic reaction under conditions that water is in a subcritical state or a supercritical state, one of the major features of the method of which resides in that the method includes the step of:
(1) collecting water from a reaction mixture after the hydrolytic reaction, adjusting a pH of the water to a pH ranging from 3.5 to 12, and feeding the collected water to the reactor; or
(2) subjecting water collected from a reaction mixture after the hydrolytic reaction to a filtration treatment with a hydrophilically treated reverse osmosis membrane, and feeding the resulting treated water as a recycled water to the reactor.

In the present invention, an unreacted water which is not used in the hydrolytic reaction of the raw material is separated and collected, and at least a part of the collected water is fed (recycled) to a reactor, so that a loss of water is reduced. Also, since the water is subjected to adjustment to a given pH or a filtration treatment with a hydrophilically treated reverse osmosis membrane, the degradation or side reactions of a glyceryl ether do not proceed during the hydrolytic reaction to an extent that would be disadvantageous from the viewpoint of the quality of the glyceryl ether, so that the worsening of the hue of the glyceryl ether can be prevented. Further, since the hydrolytic reaction is carried out under conditions that water is in a subcritical state or a supercritical state, the water used according to the stoichiometric amount effectively contributes to the reaction, so that the reaction proceeds with a high reaction selectivity even in the absence of a catalyst. Also, the removal procedure of the catalyst from the reaction product can be omitted, whereby a high-quality glyceryl ether can be efficiently produced.

The components and the like explained hereinbelow can be used alone or in a mixture of two or more kinds, so long as the exhibition of the desired effects of the present invention is not inhibited.

The glycidyl ether usable as a raw material in the present invention is a compound represented by the above-mentioned general formula (I).

In the above-mentioned formula, the hydrocarbon group having 1 to 20 carbon atoms, a part or all of the hydrogen atoms of which may be substituted with a fluorine atom, which is represented by R, includes, for example, a linear or branched alkyl group having 1 to 20 carbon atoms, a linear or branched alkenyl group having 2 to 20 carbon atoms, an aryl group having 6 to 14 carbon atoms, and the like.

Specific examples of the hydrocarbon group include, for example, a methyl group, an ethyl group, an n-propyl group, an n-butyl group, an n-pentyl group, an n-hexyl group, an n-heptyl group, an n-octyl group, an n-nonyl group, an n-decyl group, an n-dodecyl group, a tetradecyl group, a hexadecyl group, an octadecyl group, an eicosyl group, a 2-propyl group, a 2-butyl group, 2-methyl-2-propyl group, a 2-pentyl group, a 3-pentyl group, a 2-hexyl group, a 3-hexyl group, a 2-octyl group, a 2-ethylhexyl group, a phenyl group, a benzyl group, and the like. In addition, the hydrocarbon group of which hydrogen atom is substituted with a fluorine atom includes, for example, those hydrocarbon groups exemplified above of which hydrogen atom is substituted with a fluorine atom at a given position without limiting to degrees of substitution or substitution positions, including perfluoroalkyl groups, such as a nanofluorohexyl group, a hexafluorohexyl group, a tridecafluorooctyl group, a heptadecafluorooctyl group, and a heptadecafluorodecyl group; and the like.

Specific examples of the oxyalkylene group having 2 to 4 carbon atoms represented by OA include alkylene oxides, such as an oxyethylene group, an oxytrimethylene group, an oxypropylene group, and an oxybutylene group.

Here, the number of carbon atoms of the hydrocarbon group represented by R is preferably from 1 to 12, from the viewpoint of improving reaction selectivity. Also, p is preferably from 0 to 6, and more preferably 0.

Specific examples of the glycidyl ether preferably used as the raw material include n-butyl glycidyl ether, 2-methyl-2-propyl glycidyl ether, n-pentyl glycidyl ether, 2-methyl-butyl glycidyl ether, n-hexyl glycidyl ether, 2-methyl-pentyl glycidyl ether, phenyl glycidyl ether, n-octyl glycidyl ether, 2-ethyl-hexyl glycidyl ether, n-stearyl glycidyl ether, and the like.

In the present invention, the kinds of water usable in the hydrolytic reaction of the raw material are not particularly limited, so long as the exhibition of the desired effects of the present invention is not inhibited. The water includes, for example, ion-exchanged water, distilled water, water subjected to hydrophilically treated reverse osmosis filtration treatment, and the like, and those waters containing a salt or the like, such as tap water may be used without causing any disadvantages, within the range that would not impair the inherent properties of the present invention.

In addition, as the water usable in the hydrolytic reaction of the raw material in the present invention, water separated and collected from a reaction mixture after the hydrolytic reaction (reaction mixture after all or a part of the water is subjected to a hydrolytic reaction), and subjected to a pH adjustment or a filtration treatment with a reverse osmosis membrane as described below (which may be hereinafter referred to as "recycled water") is used as at least a part of the water.

The pH of the pH-adjusted water is 3.5 or more, and the upper limit is usually 12 or so. The pH is preferably from 4 to 9, and more preferably from 5 to 8, from the viewpoint of even more controlling the formation of by-products.

As an effective index for the reverse osmosis membrane of the water subjected to a filtration treatment with the above-mentioned reverse osmosis membrane, a ratio of an acid ion concentration of a recycled water to an acid ion concentration of a water collected from a reaction mixture (acid ion concentration in the recycled water / acid ion concentration of the collected water), i.e. an inhibitory ratio of acid ions, is preferably 1/2 or less, and more preferably 1/10 or less.

Here, the recycled water may be those obtained from the same reaction system, or those obtained from different systems. An embodiment of the latter includes, for example, an embodiment of Example 2 given later.

In the present invention, the amount of the recycled water used is not particularly limited, and the amount of the recycled water used in a molar ratio to the amount of water freshly fed to a reactor from the external (externally fed water) (recycled water / externally fed water) is preferably 1 or more, and more preferably 10 or more. This ratio can be said to serve as an index of a decrease in the loss of water, in which it can be generally said that the higher the value, the smaller the loss of water in the production steps. Here, the water usable in the hydrolytic reaction of the raw material may be entirely recycled water.

An embodiment of feeding a recycled water into a reactor is not particularly limited. The recycled water may be directly fed to a reactor, or the recycled water may be mixed with the raw material and/or externally fed water and the mixture may be fed to a reactor.

In the production method of the present invention, a glyceryl ether is produced by feeding a raw material and water as mentioned above to a reactor, and carrying out a hydrolytic reaction of the raw material. In the present invention, the hydrolytic reaction is carried out under conditions that the water is in a subcritical state or a supercritical state, from the viewpoint of increasing the reactivity of the raw material and water. Here, the conditions that the water is in a subcritical state refer to conditions in which the water is at a high temperature of lower than a supercritical temperature (374°C) and 200°C or higher, and a pressure equal to or higher than the saturation vapor pressure, and the conditions that the water is in a supercritical state refer to conditions in which the water is at a temperature of a supercritical temperature (374°C) or higher, and a pressure equal to or higher than a supercritical pressure (22 MPa), specifically conditions in which the water can be kept in a liquid state under a temperature of preferably from 200° to 350°C, and more preferably from 250° to 300°C, and a pressure of preferably from 1.5 to 100 MPa, and more preferably from 4.0 to 15 MPa are preferable.

The hydrolytic reaction is carried out in a reactor selected depending upon the embodiments of the production method of the present invention. The reactor is not particularly limited, so long as the reactor is capable of carrying out a hydrolytic reaction under the above-mentioned conditions, and a reaction product can be collected therefrom. For example, in a case where a hydrolytic reaction is carried out in a batch system, a vessel-type reactor, such as an autoclave, is preferably used. On the other hand, in a case where a hydrolytic reaction is carried out in a continuous system, a communicating-type tubular form such as a tubular reactor, a tower-type reactor, or a static mixer-type reactor, or a semibatch system such as a continuous agitating vessel-type reactor can be preferably used, among which a tubular reactor is especially preferably used, from the viewpoint of having markedly excellent operability and pressure tightness during a high-pressure reaction. Therefore, in the present invention, it is preferable that the hydrolytic reaction is continuously carried out with a tubular reactor. All of the above reactor s are commercially available. In addition, as the reactor, those with or without an agitation means may be used, and those with the agitation means are preferable, from the viewpoint of homogeneously progressing the reaction.

The material of the reactor usable in the present invention is not particularly limited, and a material that is generally utilizable in a chemical reaction can be arbitrarily used. Specific examples include metal materials such as steel materials such as pure iron, carbon steel, cast iron, nickel steel, austenite-based stainless steel, martensite-based stainless steel, ferrite-based stainless steel, Fe-Cr-Ni alloys, copper alloys, aluminum alloys, Ni-Cr-Fe alloys, Ni-Cu alloys, Ni-Mo-Fe-Cr alloys, cobalt alloys, titanium alloys, zirconium alloys, molybdenum, chromium, or the like; hard glass, quartz glass, porcelain ceramics, glass lining, synthetic resins, ceramic materials, and the like. Among them, in a case where a reaction is carried out under temperature conditions of a subcritical state or a supercritical state for which the corrosion of the material has been considered as a disadvantage, the metal materials such as austenite-based stainless steel, Ni-Cr-Fe alloys, and Ni-Mo-Fe-Cr alloys can be preferably utilized, and Ni-Cr-Fe alloys and Ni-Mo-Fe-Cr alloys are more preferable. Here, in the step (2) of the present invention, by subjecting a corrosive substance such as an acid, which is contained in the raw material, or a byproduct of the reaction, to a filtration treatment with a reverse osmosis membrane, the accumulation of the corrosive substance in a reaction system can be reduced, whereby the corrosion to the above-mentioned material can be reduced.

In addition, the amount of water to the raw material upon the hydrolytic reaction is not particularly limited. The amount of water, as calculated on a molar basis, is preferably from 10 to 1000 times, and more preferably from 20 to 500 times the stoichiometric amount of the raw material. Within the above range, the progress of a side reaction such as dimerization of a glycidyl ether as a raw material and a glyceryl ether that is formed is controlled, thereby increasing the reaction selectivity of the glyceryl ether. In addition, due to the peculiar phenomenon of the present reaction system, the amount of water under conditions ranging from 40 to 200 times is even more preferable, from the viewpoint of efficient productivity in the continuous reaction operations. Here, the amount of water refers to a total amount of the recycled water and the externally fed water. It is preferable that the molar ratio of the recycled water to the externally fed water is set as mentioned above.

The production method of the present invention can be also carried out in a batch system in which a raw material is fed in an amount required for a single batch, and a hydrolytic reaction is carried out in a single run, or alternatively in a continuous system in which a raw material is continuously fed, and a hydrolytic reaction is carried out. Especially, it is preferable that the method is carried out in a continuous system, having properties such as the time period needed in the rise and fall of the temperature is short, control of the reaction conditions is easier, and the reaction can efficiently be proceeded.

In a case where the method of the present invention is carried out in a batch system, it is preferable that the raw material and water are charged so that the amount of water to the raw material upon the hydrolytic reaction is within the range mentioned above. On the other hand, in a case where the method is carried out in a continuous system, it is preferable that the amount of water to the raw material is adjusted within the range mentioned above in a steady state of the reaction (i.e. a state in which the components involved in the reaction are in a steady level).

When a hydrolytic reaction is carried out, the raw material and water are individually fed to a reactor and/or previously mixed with each other and fed to the reactor. In a case where the raw material and water are fed to a reactor without previously mixing, the components are mixed in the reactor. Since a reaction system is inhomogeneous due to a chemical structure of a glycidyl ether used as a raw material, it is preferable that the mixing is carried out with an agitation means having a strong shearing force. As the agitation means, in a batch system, for example, a propeller mixer, an agi-homomixer, a homomixer, a high shearing disk turbine agitation blade, a slanted paddle agitation blade, a paddle agitation blade, or the like can be preferably used; in a continuous system, for example, a pipeline mixer, a line homomixer, a static mixer, an I. S. G. mixer, an ultrasonic mixer, a high-pressure homogenizer, a pump such as a high-shear centrifugal pump, a disper, or the like can be preferably used. Also, it is preferable that the hydrolytic reaction is proceeded under mixing conditions according to those agitation means.

In addition, although the reaction time cannot be unconditionally determined because the reaction time differs depending upon the reaction temperature, the kinds of the raw materials used, and the like. The reaction time is generally selected within the range of from 1 minute to 10 hours, from the termination of charging of raw materials and the like in a case of a batch system, or alternatively from a point at which the reaction reaches a steady state in a case of a continuous system. For example, in a case where the reaction is carried out at 200°C, the reaction time is preferably 10 minutes or so. Here, the reaction time in a continuous-type reactor refers to a time period in which a reaction mixture resides in the reactor, which is expressed by a value obtained by dividing the volume of a reactor by flow rates of the raw materials per unit time fed to the reactor.

In the present invention, since the hydrolytic reaction is carried out under conditions that water is in a subcritical state or supercritical state, the reaction may proceed in the absence of a catalyst, but an acid or alkali catalyst can be added thereto. The catalyst usable in the present invention is not particularly limited. The catalyst includes, for example, an acid, a base, a combined system of an acid and a base, and the like, which are generally usable in the hydrolytic reaction.

In a case where a catalyst is used, the amount of the catalyst used is not particularly limited, so long as the desired hydrolytic reaction efficiency of the raw material is obtained, and the amount is generally preferably from 0.01 to 10 parts by weight, and more preferably from 0.1 to 5 parts by weight, based on 100 parts by weight of the raw material.

In the manner as described above, the hydrolytic reaction of a raw material glycidyl ether is carried out. It is preferable that the separation and collection of water from a reaction mixture after the hydrolytic reaction are carried out using, for example, a separating and collecting means as described hereinbelow.

The separating and collecting means usable in the present invention refers to a means capable of collecting water and components other than water from a reaction mixture after the hydrolytic reaction. The collection of the components other than water and the collection of water may be carried out in a single unit (Embodiment 1), or each collection may be independently carried out (Embodiment 2). It is preferable to use a separating and collecting means capable of collecting water and components other than water according to Embodiment 1, from the viewpoint of procedural facilitation.

The reaction mixture after the hydrolytic reaction in the present invention has a property of allowing components other than water (including a reaction product glyceryl ether) and water to separate into two layers depending upon the chemical structure of the glycidyl ether used as a raw material. Therefore, a separating and collecting means capable of collecting water and components other than water according to Embodiment 1 includes, for example, specific gravity difference separation, membrane separation, and the like. The specific gravity difference separation includes, settling separation with an API-type oil separator, a CPI-type oil separator, a PPI-type oil separator, or the like; centrifugation with a Sharples centrifuge, a De Laval centrifuge, or the like; a wet-type cyclone, and the like. The membrane separation includes an accurate filtration membrane, an ultrafiltration membrane, a loose RO membrane (loose reverse osmosis membrane), a reverse osmosis membrane, and the like. Among them, the settling separation is preferable, because the reaction mixture is allowed to separate into two layers of components other than water (upper layer) and water (lower layer) by simply feeding the reaction mixture and allowing the reaction mixture to settle, the components other than water are collected by the collection of water, thereby making it possible to carry out the collection of the components other than water and the collection of water in a single unit.

On the other hand, a separating and collecting means capable of collecting water and components other than water according to Embodiment 2 includes, for example, a means comprising a combination of evaporation and Liebig condensation. After the reaction mixture is fed to an evaporator, the reaction mixture is heated to a temperature equal to or higher than the boiling point of water, thereby allowing water to evaporate, so that the components other than water remain in the evaporator and are collected. The evaporated water is cooled with a Liebig condenser, and collected as water. Alternatively, separation and collection may be carried out by using distillation procedures such as rectification in place of the evaporation procedures.

Here, not all the water are necessarily required to be collected from the reaction mixture. The amount of the water to be collected may be appropriately determined, and at least a part of water in the reaction mixture may be collected.

Next, in the step (1), the collected water is subjected to a pH adjustment to a given value, and thereafter fed to a reactor in which a hydrolytic reaction is carried out. Since the pH of the recycled water is lowered with the passage of time, the pH adjustment of the water is carried out with, for example, a pH adjusting agent as mentioned hereinbelow.

The above-mentioned pH adjusting agent includes, for example, ion adsorbents such as inorganic ion adsorbents and ion exchange resins; neutralizing agents such as inorganic bases and organic bases. It is preferable that the production method of the present invention is carried out in a continuous system. Using the inorganic ion adsorbent or the ion exchange resin, the lowering of the pH of water can be controlled by contacting these ion adsorbents with water; therefore, in a case where the production method of the present invention is carried out in a continuous system, it is preferable to use these adsorbents as pH adjusting agents.

The inorganic ion adsorbent includes, for example, metal composite oxides such as hydrotalcite, zeolite, and magnesium silicate; metal oxides such as alumina, silica, magnesium oxide, zinc oxide, and calcium oxide, activated charcoal; activated clay; and the like, and hydrotalcite is preferred. The ion exchange resin includes, for example, strongly basic anion exchange resins, weakly basic anion exchange resins, anion exchange membranes, anion exchange fibers, and the like, and the weakly basic anion exchange resins are preferred. Among them, the inorganic ion adsorbents are preferably used because of their excellent abilities of pH adjustment. Here, the amount of the ion adsorbent used can be determined depending upon the amount of the glycidyl ether used in the reaction. The amount of the ion adsorbent used is preferably from 0.01 to 20 parts by weight, and more preferably from 0.1 to 10 parts by weight, based on 100 parts by weight of the glycidyl ether.

The neutralizing agents include, for example, inorganic bases such as metal carbonates such as sodium carbonate, potassium carbonate, calcium carbonate, magnesium carbonate, sodium hydrogencarbonate, and potassium hydrogencarbonate, metallic hydroxides such as sodium hydroxide, potassium hydroxide, calcium hydroxide, magnesium hydroxide, and aluminum hydroxide, and metallic phosphates such as calcium phosphate and calcium hydrogenphosphate; and organic bases such as ammonia, aniline, ethylamine, diethylamine, triethylamine, butylamine, and pyridine.

The method for adjusting a pH includes, for example, a method including the steps of packing a column with an inorganic ion adsorbent or an ion exchange resin as mentioned above, and allowing the separated and collected water to pass through the column. Here, as a means of treating the separated and collected water in the column, the treatment may be carried out in either a continuous system or a batch system without having any disadvantages. In a continuous system, the separated and collected water may be treated in series, or treated in circulation via a temporary storage tank or the like. Alternatively, the method includes, for example, a method of adding a neutralizing agent as mentioned above to the separated and collected water, and adjusting a mixed solution to a desired pH. The pH adjustment may be carried out by any one of these methods, or a combination thereof. In a case where the production method of the present invention is carried out in a continuous system, the former method of using an adsorbent or the like is more preferable because the pH adjustment of water is facilitated.

Here, in the present invention, the pH of water is a value measured at an ordinary temperature and a normal pressure. The method of measuring the pH in the present invention is not particularly limited. The method includes, for example, an indicator method, a metal electrode method with a hydrogen electrode, an antimony electrode or the like, and a glass electrode method. Here, the pH measurement may be technologically carried out within the numerical range as calculated at an ordinary temperature and normal pressure in accordance with the temperature and pressure changes.

Here, in a case where water is collected by a separating and collecting means which is capable of collecting water and components other than water according to the above-mentioned Embodiment 2, the pH of the collected water may be 3.5 or higher in some cases. In the application of the step (1), in such a case, the collection of the water and the adjustment of the pH of water are assumed to be carried out at the same time. In addition, even if the pH of the collected water is already in a pH range from 3.5 to 12 , in a case where the method goes through pH adjustment procedures as mentioned above, the pH adjustment is considered to be carried out in the present invention.

On the other hand, in the step (2), the collected water is subjected to a filtration treatment with a hydrophilically treated reverse osmosis membrane, and thereafter filtered water is fed to a reactor in which a hydrolytic reaction is carried out. The filtration treatment is carried out using a hydrophilically treated reverse osmosis membrane as mentioned below.

A material of the reverse osmosis membrane includes an acetic acid cellulose-based membrane, an aromatic polyamide-based membrane, a polybenzimidazolone-based membrane, a polyacrylonitrile-based membrane, a polysulfone-based membrane, and composite membranes thereof, and the like. According to the present invention, a low-contamination membrane in which a surface of these materials is subjected to a hydrophilic treatment is used

As a shape of the reverse osmosis membrane, those having a spiral form, a tubular form, a capillary form, a hollow thread form, a flat membrane form, or the like have been actually used, any of which can be used in the present invention.

As the reverse osmosis membrane, commercially available products sold from TORAY INDUSTRIES, INC., NITTO DENKO CORPORATION, and the like. In a case where the removal of chlorine ions is used as an index, those having an inhibition ratio of 50% or more are preferable, those having an inhibition ratio of 90% or more are more preferable, and those having an inhibition ratio of 99% or more are even more preferable.

The operating pressure for carrying out reverse osmosis filtration is preferably 0.1 MPa or more, from the viewpoint of making it possible to quicken a permeation rate of a liquid, shorten the treatment time, and make the membrane area compact, and the operating pressure is preferably 10 MPa or less, from the viewpoint of not requiring pressure tightness of the apparatus, and facilitating management and maintenance. From these viewpoints, the operating pressure for carrying out reverse osmosis filtration is preferably from 0.1 to 10 MPa, and more preferably from 0.5 to 5 MPa.

In the filtration treatment with a reverse osmosis membrane, the filtration property is influenced by a pH of the water subjected to a filtration treatment. In general, the chemical reaction using a subcritical water or supercritical water tends to be acidic. Therefore, in the present invention, the pH adjustment of the water collected may be carried out before the filtration treatment, from the viewpoint of removing an acidic substance in the water collected from the reaction mixture. In addition, the pH adjustment of the water collected from the reaction mixture may be carried out before or after the filtration treatment, from the viewpoint of preventing the corrosion of the apparatus. In this case, the pH of the adjusted water is preferably from 5 to 10, and more preferably from 7 to 9. Here, the pH adjustment of water is carried out with, for example, a pH adjusting agent mentioned above.

The method of adjusting a pH may be carried out in any one of, for example, a continuous system, a batch system, a semi-batch system, or the like without causing any disadvantages. In the continuous system, the separated and collected water may be subjected to treatments in series, or may be subjected to treatments in circulation via a temporary storage tank or the like. Alternatively, the method includes, for example, a method including the steps of adding a neutralizing agent as mentioned above to the separated and collected water, and adjusting to a desired pH. The pH adjustment may be carried out by any one of these methods, or a combination thereof.

In a case where the production method of the present invention is carried out in a continuous system in the step (1), as exemplified in Example 1 given later, the method includes an embodiment in which a series of procedures including the steps of separating and collecting water from a reaction mixture discharged from a reactor, allowing the water to pass through a column packed with, for example, the above-mentioned adsorbent or the like to adjust a pH of the water, and feeding the water again to the reactor via a circulation line in order to be used in a further hydrolytic reaction, are carried out in the same apparatus. Also, in a case where the method is carried out in a continuous system in the step (2), as exemplified in Example 3 given later, the method includes an embodiment in which a series of procedures including the steps of separating and collecting water from a reaction mixture discharged from a reactor, thereafter subjecting the water to a filtration treatment with a reverse osmosis filter, and feeding the treated water again to the reactor via a circulation line in order to be used in a further hydrolytic reaction, are carried out in the same apparatus. On the other hand, in a case where the method is carried out in a batch system in the step (1), as exemplified in Example 2 given later, the method includes an embodiment in which a series of procedures including the steps of separating and collecting water from a reaction mixture, for example, adding a neutralizing agent mentioned above to the collected water to adjust a pH of the water, and feeding the water after the pH adjustment to the reactor, are carried out in the same apparatus or different apparatuses. Here, the embodiment of feeding a recycled water to a reactor is not particularly limited, and the recycled water may be directly fed to a reactor, or the recycled water may be mixed with a raw material and/or externally fed water, and the mixture is fed to a reactor.

A reaction product glyceryl ether is obtained as a component other than the above water. The component other than water is usually usable for a glyceryl ether directly. For example, in a case where a catalyst is used, it is preferable that the glyceryl ether is further purified by means of evaporation, distillation, extraction, precision filtration, adsorption, or the like, in accordance with, for example, a known method.

As described above, a glyceryl ether is obtained. In a case where the water collected in the manner mentioned above is not subjected to a pH adjustment, or not subjected to a filtration treatment with a reverse osmosis membrane, the pH of the water usable in the hydrolytic reaction of a raw material is lowered with the passage of time, or the formation of by products is increased; consequently, the degradation and side reactions of the glyceryl ether are likely to proceed, and the elution of the metal is caused depending upon the reactor used, whereby the resulting glyceryl ether has a worsened hue or is accompanied with an irritating odor. According to the production method of the present invention, even when a recycled water is used in a hydrolytic reaction for a long period of time, a high-quality glyceryl ether can be obtained by suppressing the generation of various phenomena as mentioned above.

Here, in the present invention, the hue of the glyceryl ether is determined by Gardner method (as prescribed in JIS K 0071-2). The glyceryl ether obtainable by the production method of the present invention has a hue of preferably 4 or less, and more preferably 3 or less.

In addition, the pH adjustment or the filtration treatment with a reverse osmosis membrane of the water collected from a reaction mixture in the production method of the present invention can be understood as a means of preventing coloration of a glyceryl ether, in a case of viewing from the side of the glyceryl ether obtainable by the method. Therefore, as one embodiment of the present invention, a method for preventing coloration of a glyceryl ether, produced by the steps of (A) feeding a compound represented by the above-mentioned general formula (I), and water to a reactor, and subjecting the compound to a hydrolytic reaction under conditions that water is in a subcritical state or a supercritical state, and (B) collecting water from the reaction mixture after the hydrolytic reaction, and feeding the collected water to the reactor, wherein the method for preventing coloration of a glyceryl ether comprises the step, in the step (B), of (B-1) adjusting a pH of the water to 3.5 or more, and feeding the collected water to the reactor; or (B-2) subjecting the water to a filtration treatment with a hydrophilically treated reverse osmosis membrane, and feeding the resulting treated water as a recycled water to the reactor is provided. Regarding the procedures of each step in the method, the step (B-1) may be carried out in accordance with the description on the above-mentioned step (1), and the step (B-2) may be carried out in accordance with the description on the above-mentioned step (2).

### EXAMPLES

### Example 1 (Reference Example)

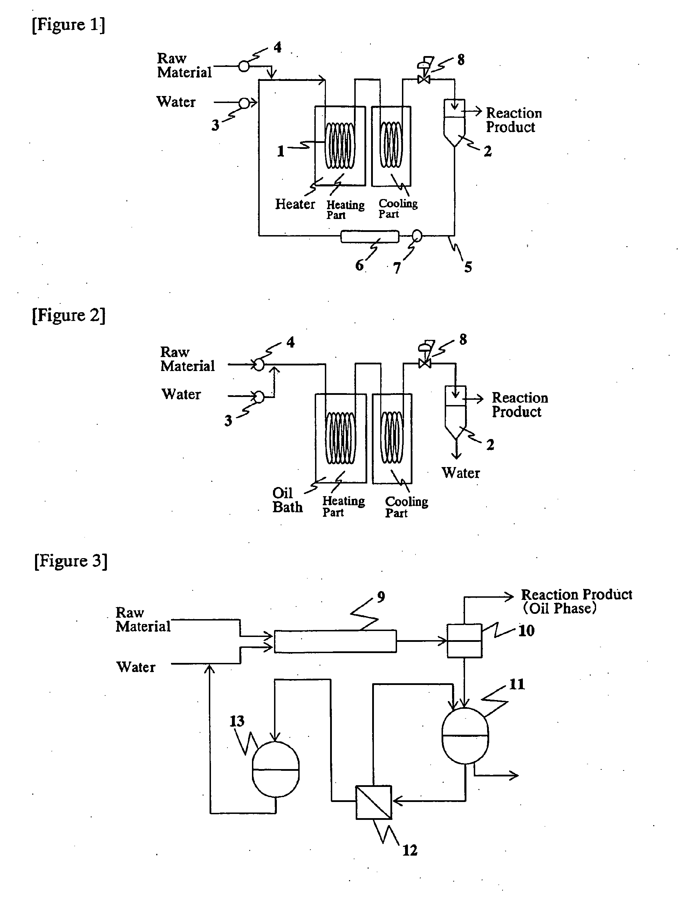

A reaction apparatus shown in Figure 1 was used. The apparatus shown in Figure 1 comprises a tubular reactor 1, a separating and collecting part 2, a water-feeding part 3, and a raw material-feeding part 4. Each of the water-feeding part 3 and the raw material-feeding part 4 is connected to the tubular reactor 1, and the tubular reactor 1 is connected to the separating and collecting part 2 via a water-circulation line 5. In the separating and collecting part 2, a settling separator is used, and the water-circulation line 5 is equipped with a water-treating part 6 and a water-circulation pump 7. In the water-treating part 6, as an inorganic ion adsorbent, a column made of SUS steel packed with 200 g of KYOWARD 1015 [hydrotalcite, commercially available from Kyowa Chemical Industries] was used.

Upon the beginning of the production of a glyceryl ether, a separating and collecting part 2 was charged with 13 kg of ion-exchanged water. Continuously fed to a tubular reactor 1 (tube diameter: 16 mm, tube length: 11 m, and material: SUS316) were a raw material 2-ethyl-hexyl glycidyl ether at a rate of 50.1 g/minute, and ion-exchanged water (initial pH 6.8) at a rate of 28.6 g/minute from a raw material-feeding part 4 and a water-feeding part 3, and the water from the separating and collecting part 2 at a rate of 464.3 g/minute via a water-circulation line 5, respectively. A reaction mixture was introduced into the separating and collecting part 2 through a back pressure valve 8. The tubular reactor 1 was heated with a heater so that the temperature of the internal fluid (reaction temperature) reached 275°C. On the other hand, the pressure inside the tubular reactor 1 (reaction pressure) was adjusted to 8 MPa. In the tubular reactor 1, the hydrolytic reaction of the raw material was carried out under the temperature and pressure conditions, i.e. under conditions that the water was in a subcritical state. Here, the amount of water to the raw material in the steady state of the reaction, on a molar basis, was 100 times its stoichiometric amount. After the hydrolytic reaction, the reaction mixture was cooled by a cooling part (50°C), and thereafter the reaction mixture reached the separating and collecting part 2. In the separating and collecting part 2, the reaction mixture was allowed to separate into two layers, and water was collected as a lower layer. The water was allowed to pass through a water-treating part 6 via a water-circulation line 5, to adjust a pH of the water at all times, and thereafter the water was fed to the tubular reactor 1.

By the above procedures, a glyceryl ether was produced. Every four hours from the feeding of the glycidyl ether and the beginning of the hydrolytic reaction, a reaction product was collected until after 28 hours. The reaction selectivity and degradation ratio of the glyceryl ether, as calculated from the gas chromatogram of the reaction product obtained during a period from the beginning of the collection to the feeding of 300 g of 2-ethyl-hexyl glycidyl ether to the tubular reactor 1, and the hue of the glyceryl ether are shown in Table 1. The pH (measured at ambient temperature and normal pressure, according to a glass electrode method) of the water collected from the reaction mixture after passing through a water-treating part 6 while the reaction product was collected is also shown together in Table 1.

Here, the reaction selectivity was calculated by: a formation ratio of the formed glyceryl ether / a conversion ratio of the fed glycidyl ether x 100. In addition, the degradation ratio was calculated by the formation ratio of the degraded product of a glyceryl ether. The hue was determined by Gardner method (as prescribed in JIS K 0071-2).

### Comparative Example 1

The same apparatus as shown in Figure 1 was used, except that it was set in this example that the collected water was directly introduced into the tubular reactor 1 from a separating and collecting part 2 via a water-circulation line 5 without setting a water-treated part 6.

Upon the beginning of the production of a glyceryl ether, the separating and collecting part 2 was charged with 13 kg of ion-exchanged water. Continuously fed to a tubular reactor 1 were a raw material 2-ethyl-hexyl glycidyl ether at a rate of 50.1 g/minute, and ion-exchanged water (initial pH 6.8) at a rate of 28.6 g/minute from a raw material-feeding part 4 and a water-feeding part 3, and the water from the separating and collecting part 2 at a rate of 464.3 g/minute via a water-circulation line 5, respectively. A reaction mixture was introduced into the separating and collecting part 2 through a back pressure valve 8. The tubular reactor 1 was heated with a heater so that the temperature of the internal fluid (reaction temperature) reached 275°C. On the other hand, the pressure inside the tubular reactor 1 (reaction pressure) was adjusted to 8 MPa. In the tubular reactor 1, the hydrolytic reaction of the raw material was carried out under the temperature and pressure conditions, i.e. under conditions that the water was in a subcritical state. After the hydrolytic reaction, the reaction mixture was cooled by a cooling part (50°C), and thereafter reached the separating and collecting part 2. In the separating and collecting part 2, the reaction mixture was allowed to separate into two layers, and water was collected as a lower layer. The water was fed to the tubular reactor 1 at all times via a water-circulation line 5.

By the above procedures, a glyceryl ether was produced. Every four hours from the feeding of the glycidyl ether and the beginning of the hydrolytic reaction, a reaction product was collected until after 16 hours. The reaction selectivity and degradation ratio of the glyceryl ether, as calculated from the gas chromatogram of the reaction product obtained while feeding 300 g of 2-ethyl-hexyl glycidyl ether to the tubular reactor 1 since the collecting was began, and the hue of the glyceryl ether are shown in Table 1. The pH of the water collected from the reaction mixture and in a state of the lower layer of the separating and collecting part 2 is also shown together in Table 1.

Here, the reaction selectivity, the degradation ratio, and the hue of the glyceryl ether and the pH of the water were calculated or determined in the same manner as in Example 1.

**[Table 1]**

| | Operating Time | pH of Water | Reaction Selectivity | Degradation Ratio | Hue |
|---|---|---|---|---|---|
| | (h) | | (%) | (%) | (G) |
| Ex. 1 | 4 | 6.2 | 97.8 | 0.6 | 2 |
| | 8 | 5.2 | 97.6 | 0.6 | 2 |
| | 12 | 5.0 | 97.3 | 0.6 | 2 |
| | 16 | 5.0 | 97.8 | 0.6 | 2 |
| | 20 | 4.9 | 98.1 | 0.6 | 2 |
| | 24 | 5.0 | 98.2 | 0.6 | 2 |
| | 28 | 5.0 | 97.8 | 0.6 | 2 |
| Comp. Ex. 1 | 4 | 3.4 | 97.6 | 2.4 | 8 |
| | 8 | 3.3 | 97.4 | 2.6 | 9 |
| | 12 | 3.2 | 97.2 | 2.8 | 9 |
| | 16 | 3.2 | 97.1 | 3.1 | 10 |

In Example 1, a hydrolytic reaction was carried out by using an inorganic ion adsorbent as a pH adjusting agent of water, the pH of the water collected from the reaction mixture was continuously adjusted after the hydrolytic reaction, and thereafter the adjusted water was circulated into the reactor. As a result, as shown in Table 1, even when operating for a long period of time, a high reaction selectivity of the glyceryl ether was shown, and the degradation ratio of the glyceryl ether was low, and the worsening of the hue was not found.

On the other hand, a hydrolytic reaction was carried out without using a pH adjusting agent of water, and the water collected from a reaction mixture was circulated directly to the reactor. As a result, as shown in Table 1, the reaction selectivity tends to be decreased with the passage of the operating time, so that the degradation ratio was evidently higher than those of Example 1. Also, the marked worsening of the hue was found.

### Example 2 (Reference Example)

A reaction apparatus shown in Figure 2 was used. The apparatus shown in Figure 2 comprises a tubular reactor 1, a separating and collecting part 2, a water-feeding part 3, a raw material-feeding part 4, and a back pressure valve 8. Each of the water-feeding part 3 and the raw material-feeding part 4 is connected to the tubular reactor 1, and the tubular reactor 1 is connected to the separating and collecting part 2. As a separating and collecting part 2, a settling separator was used.

To the water collected and separated after 20 hours from the feeding the glycidyl ether and the beginning of the hydrolytic reaction in Comparative Example 1, was added sodium hydroxide as a neutralizing agent to adjust the pH of the collected water to 6.0. Continuously fed to a tubular reactor 1 were the collected water at a rate of 15.9 mL/minute from a water-feeding part 3, and 2-ethyl-hexyl glycidyl ether at a rate of 1.8 mL/minute from a raw material-feeding part 4, respectively. A reaction mixture was introduced into the separating and collecting part 2 through a back pressure valve 8. Here, the temperature of the internal fluid inside the tubular reactor 1 was maintained at 280°C with an oil bath. On the other hand, the pressure inside the tubular reactor 1 was adjusted to 8 MPa. In the tubular reactor 1, the hydrolytic reaction of the raw material was carried out under the temperature and pressure conditions, i.e. under conditions that the water was in a subcritical state. Here, the amount of water to the raw material in the steady state of the reaction, on a molar basis, was 100 times its stoichiometric amount. After the hydrolytic reaction, the reaction mixture was cooled by a cooling part (50°C), and thereafter the reaction mixture reached the separating and collecting part 2. In the separating and collecting part 2, the reaction mixture was allowed to separate into two layers, and the reaction product was collected as an upper layer. By the above procedures, the reaction product was collected after 2 hours from a point where the reaction components were in a steady state. The selectivity for the glyceryl ether as calculated from the gas chromatogram of the reaction product obtained during a period from the beginning of the collecting to the feeding of 20 g of 2-ethyl-hexyl glycidyl ether was 98.3%. In addition, the degradation ratio of the glyceryl ether was 0.7%.

In Example 2, by adjusting the pH of the water separated and collected after the hydrolytic reaction of Comparative Example 1, and using the water in the hydrolytic reaction, a high reaction selectivity of the glyceryl ether was obtained, and the degradation ratio of the glyceryl ether could also be controlled to a low level. In addition, the worsening of the hue was not found.

### Examples 3 and 4

A reaction apparatus shown in Figure 3 was used. The apparatus shown in Figure 3 comprises a tubular reactor 9, a separating and collecting part 10, a storage tank 11, a reverse osmosis filter 12, and a storage tank 13.

Upon the beginning of the production of a glyceryl ether, continuously fed to a tubular reactor 9 were 2-ethylhexyl glycidyl ether (purity: 99.8% or more) at a rate of 0.108 kg/minute, and ion-exchanged water at a rate of 1.075 kg/minute, and the components were reacted continuously at 270°C. The pressure inside the tubular reactor 9 (reaction pressure) was adjusted to 8.0 MPa. In the tubular reactor 9, the hydrolytic reaction of the raw material was carried out under the temperature and pressure conditions, i.e. under conditions that the water was in a subcritical state. Here, the amount of water to the raw material in the steady state of the reaction, on a molar basis, was 100 times its stoichiometric amount. After the hydrolytic reaction, the reaction mixture was cooled by a cooling part (70°C), and thereafter the reaction mixture reached the separating and collecting part 10. In the separating and collecting part 10, the reaction mixture was allowed to separate into two layers by settling separation, and a reaction product (glyceryl ether) was isolated as an upper layer oil phase. On the other hand, a lower layer aqueous phase was collected at a storage tank 11 at a rate of 1.054 kg/minute, and the collected aqueous phase was continuously collected in the storage tank 13, directly in Example 3, or as a treated water in Example 4, obtained by adjusting the collected aqueous phase to a pH of 9.0, feeding the aqueous phase to a reverse osmosis filter 12 (commercially available from NITTO DENKO CORPORATION, LF10-D2, aromatic polyamide-based, hydrophilically treated reverse osmosis membrane, 1.8 m², 2 inch spiral form, chlorine ion inhibitory ratio: 99.5%) at a pressure of 2.0 MPa and a flow rate of 5.0 L/min, and subjecting the aqueous phase to a filtration treatment with the reverse osmosis membrane at a permeation rate of 1.000 kg/min. The chlorine ion concentrations in this case were such that the water before the permeation was 1.15 mmol/kg, and the treated water after the permeation was 0.001 mmol/kg, thereby showing an inhibitory ratio of 99.9%. The concentrated water not permeated through the reverse osmosis membrane was again returned to a storage tank 11, and the storage tank 11 was controlled by discharging the water so that a liquid surface would be leveled. The treated water collected in the storage tank 13 was used as a recycled water, and the recycled water was supplementarily continuously fed to the tubular reactor 9 at a rate of 1.000 kg/min in place of controlling the feed amount of ion-exchanged water to 0.075 kg/min.

By the above procedures, a glyceryl ether was continuously produced. The pH of the recycled water after 9 hours from the feeding of the glycidyl ether and the beginning of the hydrolytic reaction was 4.2 in Example 3. Also, the acid ion concentrations in the recycled water (total concentration of hydrochloric acid and formic acid) were 0.25 mmol/kg in Example 3, and 0.02 mmol/kg in Example 4. Further, a Gardner hue of the reaction product was determined by Gardner method (as prescribed in JIS K 0071-2). The results are shown in Table 2.

### Comparative Example 2

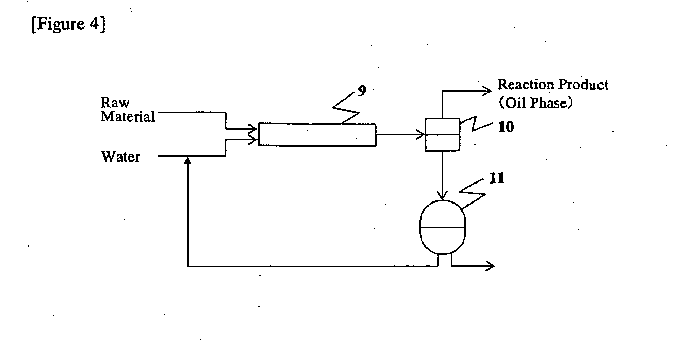

A reaction apparatus shown in Figure 4 was used. The apparatus shown in Figure 4 comprises a tubular reactor 9, a separating and collecting part 10, and a storage tank 11.

The raw material was allowed to react in the same manner as in Example 3, and the resulting reaction mixture was allowed to separate into an oil phase containing glyceryl ether and an aqueous phase by subjecting the reaction mixture to a settling separation in the separating and collecting part 10. The aqueous phase was collected in a storage tank 11, and used as a recycled water, and the recycled water was supplementarily fed to the tubular reactor 9 at a rate of 1.000 kg/minute in place of controlling the feed rate of the ion-exchanged water to 0.075 kg/minute, and controlled by discharging the water so that the liquid surface of the storage tank 11 was leveled.

The pH of the recycled water after 9 hours from feeding the glycidyl ether and beginning the hydrolytic reaction was 3.3, and the acid ion concentration of the recycled water was 2.72 mmol/kg. The results of the Gardner hue of the reaction product are shown in Table 2.

**[Table 2]**

| | Filtration Treatment with hydrophilically treated Reverse Osmosis Membrane | Gardner Hue of Reaction Product |
|---|---|---|
| Ex.3 | Positive | 1 |
| Ex. 4 | Positive | 1 |
| Comp. Ex. 2 | Negative | 12 |

From the above results, the reaction products maintained the hue of 1 in Examples 3 and 4, showing no changes in the hue. On the other hand, in Comparative Example 2 where the water is not subjected to a filtration treatment with a reverse osmosis membrane, the hue undergoes a change to 12 a brown color; therefore, it can be seen that the glyceryl ether having an excellent hue can be efficiently obtained by the filtration treatment with a reverse osmosis membrane. Here, the yield and purity of the glyceryl ethers in Examples 3 and 4, and Comparative Example 2 were of the same level, each having a yield of 99% and a purity of 97%.

### INDUSTRIAL APPLICABILITY

According to the present invention, a high-quality glyceryl ether which is usable for a solvent, an emulsifying agent, a dispersant, a detergent, a foaming agent, or the like can be provided.

### BRIEF DESCRIPTION OF THE DRAWINGS

[Figure 1] An apparatus schematic view showing one example of the apparatus preferably used in carrying out the production method of the present invention.
[Figure 2] An apparatus schematic view showing one example of the apparatus preferably used in carrying out the production method of the present invention.
[Figure 3] A schematic view of an apparatus used in Examples 3 and 4.
[Figure 4] A schematic view of an apparatus used in Comparative Example 2.

### EXPLANATION OF NUMERALS

- 1: tubular reactor
- 2: separating and collecting part
- 3: water-feeding part
- 4: raw material-feeding part
- 5: water-circulation line
- 6: water-treating part
- 7: water-circulation pump
- 8: back pressure valve
- 9: tubular reactor
- 10: separating and collecting part
- 11: storage tank
- 12: reverse osmosis filter
- 13: storage tank

## Claims

1. A method for producing a glyceryl ether, comprising feeding a compound represented by the general formula (I): 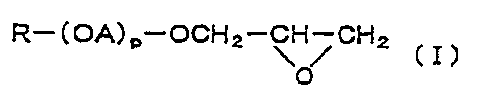 wherein R is a hydrocarbon group having 1 to 20 carbon atoms, a part or all of the hydrogen atoms of which may be substituted with a fluorine atom; OA, which may be identical or different, is an oxyalkylene group having 2 to 4 carbon atoms; and p is the number of from 0 to 20,
and water to a reactor, and subjecting the compound to a hydrolytic reaction under conditions that water is in a subcritical state or a supercritical state; and
wherein the method includes the step of:
(1) collecting water from a reaction mixture after the hydrolytic reaction, adjusting a pH of the water to a pH ranging from 3.5 to 12 , and feeding the collected water to the reactor; or
(2) subjecting water collected from a reaction mixture after the hydrolytic reaction to a filtration treatment with a hydrophilically treated reverse osmosis membrane, and feeding the resulting treated water as a recycled water to the reactor.

2. The method according to claim 1, wherein the pH of water is adjusted with an inorganic ion adsorbent.

3. The method according to claim 1, wherein an acid ion concentration in the recycled water is controlled to 1/2 or less of an acid ion concentration of the water collected from the reaction mixture after the hydrolytic reaction.

4. The method according to claim 1, wherein a pH is adjusted before or after the filtration treatment in the above step (2).

5. The method according to any one of claims 1 to 4, wherein an amount of water to the compound represented by the general formula (I) in the hydrolytic reaction is 10 to 1000 times its stoichiometric amount, when calculated on a molar basis.

6. The method according to any one of claims 1 to 4, wherein the hydrolytic reaction is carried out within a temperature range of from 200° to 350°C.

7. The method according to any one of claims 1 to 4, wherein the hydrolytic reaction is continuously carried out.

8. The method according to any one of claims 1 to 4, wherein the reaction mixture after the hydrolytic reaction is subjected to settling separation, thereby collecting water therefrom.

## Patentansprüche

1. Verfahren zur Herstellung eines Glycerylethers, das das Zuführen einer Verbindung der Formel (I): 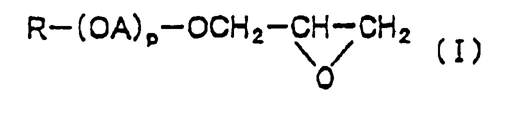 worin R eine Kohlenwasserstoffgruppe mit 1 bis 20 Kohlenstoffatomen ist, bei der ein Teil oder alle der Wasserstoffatome durch Fluoratome ersetzt sein können; OA, die gleich oder unterschiedlich sein können, eine Oxyalkylengruppe mit 2 bis 4 Kohlenstoffatomen ist; und p eine Zahl von 0 bis 20 ist, und Wasser zu einem Reaktor, und Durchführen einer Hydrolysereaktion mit der Verbindung unter Bedingungen, so dass Wasser in einem subkritischen Zustand oder einem super-kritischen Zustand ist; und
worin das Verfahren die Schritte des:
(1) Einsammelns von Wasser aus der Reaktionsmischung nach der Hydrolysereaktion, Einstellens des pH-Werts des Wassers auf einen pH-Wert im Bereich von 3,5 bis 12 und Zuführens des eingesammelten Wassers zum Reaktor; oder
(2) Durchführens einer Filtrationsbehandlung des aus der Reaktionsmischung nach der Hydrolysereaktion eingesammelten Wassers mit einer hydrophil behandelten Umkehrosmosemembran, und Zuführen des resultierenden behandelten Wassers als wiederaufbereitetes Wasser zum Reaktor.

2. Verfahren gemäß Anspruch 1, worin der pH-Wert des Wassers mit einem anionischen Ionenadsorber eingestellt wird.

3. Verfahren gemäß Anspruch 1, worin die Säureionenkonzentration im wiederaufbereiteten Wasser auf 1/2 oder weniger der Säureionenkonzentration des aus der Reaktionsmischung nach der Hydrolysereaktion eingesammelten Wassers eingestellt wird.

4. Verfahren gemäß Anspruch 1, worin der pH vor oder nach der Filtrationsbehandlung im obigen Schritt (2) eingestellt wird.

5. Verfahren gemäß irgendeinem der Ansprüche 1 bis 4, worin die Menge des Wassers zur Verbindung der Formel (I) in der Hydrolysereaktion das 10- bis 1.000-fache ihrer stöchiometrischen Menge ist, berechnet auf einer molaren Basis.

6. Verfahren gemäß irgendeinem der Ansprüche 1 bis 4, worin die Hydrolysereaktion innerhalb eines Temperaturbereichs von 200°C bis 350°C durchgeführt wird.

7. Verfahren gemäß irgendeinem der Ansprüche 1 bis 4, worin die Hydrolysereaktion kontinuierlich durchgeführt wird.

8. Verfahren gemäß irgendeinem der Ansprüche 1 bis 4, worin die Reaktionsmischung nach der Hydrolysereaktion einer Absetztrennung unterzogen wird, wodurch das Wasser aus ihr eingesammelt wird.

## Revendications

1. Procédé pour produire un éther glycérylique, comprenant l'alimentation d'un composé représenté par la formule générale (I) : 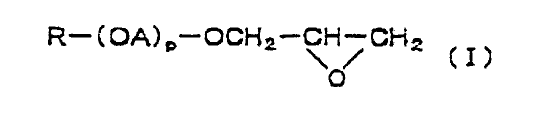 dans laquelle R est un groupe hydrocarboné ayant 1 à 20 atomes de carbone, dont une partie ou la totalité des atomes d'hydrogène peuvent être substitués par un atome de fluor ; OA, qui peuvent être identiques ou différents, est un groupe oxyalkylène ayant 2 à 4 atomes de carbone ; et p est le nombre de 0 à 20, et d'eau dans un réacteur, et la soumission du composé à une réaction d'hydrolyse dans des conditions où l'eau est dans un état sous-critique ou un état supercritique ; et
dans lequel le procédé inclut l'étape de :
(1) recueil d'eau à partir d'un mélange réactionnel après la réaction d'hydrolyse, ajustement d'un pH de l'eau à un pH allant de 3,5 à 12, et alimentation de l'eau recueillie vers le réacteur ; ou
(2) soumission de l'eau recueillie à partir d'un mélange réactionnel après la réaction d'hydrolyse à un traitement de filtration avec une membrane d'osmose inverse traitée par voie hydrophile, et alimentation de l'eau traitée résultante comme eau recyclée vers le réacteur.

2. Procédé selon la revendication 1, dans lequel le pH de l'eau est ajusté avec un adsorbant d'ions inorganique.

3. Procédé selon la revendication 1, dans lequel une concentration en ions acides dans l'eau recyclée est contrôlée jusqu'à la moitié ou moins d'une concentration en ions acides de l'eau recueillie à partir du mélange réactionnel après la réaction d'hydrolyse.

4. Procédé selon la revendication 1, dans lequel un pH est ajusté avant ou après le traitement de filtration à l'étape (2) ci-dessus.

5. Procédé selon l'une quelconque des revendications 1 à 4, dans lequel une quantité d'eau par rapport au composé représenté par la formule générale (I) dans la réaction d'hydrolyse est de 10 à 1 000 fois sa quantité stoechiométrique, lorsqu'elle est calculée sur une base molaire.

6. Procédé selon l'une quelconque des revendications 1 à 4, dans lequel la réaction d'hydrolyse est réalisée dans une plage de températures de 200° à 350°C.

7. Procédé selon l'une quelconque des revendications 1 à 4, dans lequel la réaction d'hydrolyse est réalisée de manière continue.

8. Procédé selon l'une quelconque des revendications 1 à 4, dans lequel le mélange réactionnel après la réaction d'hydrolyse est soumis à une séparation par décantation, recueillant par ce moyen l'eau de celui-ci.
